# FASCICULE DE BREVET EUROPEEN

(11) **EP 3 277 237 B1**
(45) Date de publication et mention de la délivrance du brevet: **07.05.2025**
(21) Numéro de dépôt: 16719446.3
(22) Date de dépôt: 25.03.2016
(51) Int. Cl.: A61F 9/007

(54) **IMPLANT OPHTALMOLOGIQUE D'INTERPOSITION**
INTERPOSITIONELLES OPHTHALMOLOGISCHES IMPLANTAT
INTERPOSITIONAL OPHTHALMOLOGICAL IMPLANT

(30) Priorité: 31.03.2015 FR 1552732
(43) Date de publication de la demande: 07.02.2018
(73) Titulaire: CILIATECH, 74330 Epagny Metz-Tessy (FR)
(72) Inventeur: SOURDILLE Philippe, 86800 Liniers (FR)
(74) Mandataire: Ipsilon
(86) Numéro de dépôt international: PCT/FR2016/050696
(87) Numéro de publication internationale: WO 2016/156727

(56) Documents cités:
- EP-A1- 0 766 544
- US-A- 4 521 210
- US-A- 4 521 210
- US-A1- 2004 015 140
- US-A1- 2004 092 856
- US-A1- 2004 254 521

## Description

L'invention est relative à un implant ophtalmologique d'interposition destiné à maintenir séparés définitivement l'un de l'autre la sclère et le corps ciliaire pour abaisser la pression intra oculaire (PIO).

La pression intra-oculaire résulte d'un équilibre entre sa sécrétion par le corps ciliaire et son écoulement à travers les mailles du trabéculum cornéo-scléral par le canal de Schlemm et ses émonctoires jusqu'aux veines aqueuses et à la circulation générale. Une fraction représentant 10 à 15% de cet écoulement se fait directement à travers le trabeculum ciliaire entre la sclère et le corps ciliaire, c'est ce qu'on appelle l'écoulement uvéo-scléral. Les fibres longitudinales du muscle ciliaire, particulièrement au cours de l'accomodation, jouent un rôle de mise en tension des mailles trabeculaires, ce qui facilite l'écoulement uvéo-scléral de l'humeur aqueuse.

Dans le glaucome l'écoulement de l'humeur aqueuse est diminué au niveau du trabéculum, ce qui entraine dans la majorité des cas une augmentation de la pression intra oculaire (PIO). L'abaissement de cette PIO est donc l'élément déterminant du traitement médical et/ou chirurgical du glaucome. Le traitement chirurgical repose sur deux possibilités : diminuer la production d'humeur aqueuse élaborée par le corps ciliaire (cyclo affaiblissement) ou augmenter l'écoulement d'humeur aqueuse en la dérivant. Cette dérivation est effectuée de différentes façons :
- en faisant communiquer directement la chambre antérieure et l'espace supra choroidien (cyclodialyse et ses dérivés techniques), mais l'effet obtenu est le plus souvent transitoire et insuffisant. En désinsérant l'insertion du muscle ciliaire à l'éperon scléral cette technique d'implantation supprime le mécanisme physiologique de l'écoulement uvéo-scléral dans la zone chirurgicale. De plus la fibrose post-opératoire s'étend au-delà de cette zone. Il est par ailleurs connu que la partie de l'implant située dans la chambre antérieure peut toucher, même de manière intermittente ; l'endothélium cornéen, entrainant de ce fait un risque significatif d'œdème cornéen évolutif.
- en incisant, à partir de la chambre antérieure, le trabéculum jusqu'au canal de Schlemm pour court-circuiter l'obstacle trabéculaire. Cette intervention peut être complétée par la pose, dans le canal d'un stent ouvert vers la chambre antérieure pour maintenir un accès direct permanent de l'humeur aqueuse au canal. Là aussi, les résultats sont souvent partiels et temporaires, et n'exonèrent pas de la continuation ou de la reprise du traitement médical.

La chirurgie filtrante est la plus fréquemment utilisée et cherche à dériver l'humeur aqueuse sous la conjonctive pour obtenir la baisse de pression nécessaire. Elle peut créer un orifice permanent de pleine épaisseur dans le trabéculum sous un volet scléral : la trabéculectomie. Elle peut aussi laisser en place la partie interne du trabéculum, ce qu'on appelle la chirurgie non perforante du trabéculum (sclérectomie profonde, viscocanalostomie). Cependant, la chirurgie filtrante connait des complications liées à l'insuffisance de la filtration par fibrose de la bulle de filtration (la bulle de filtration est présente entre la sclère et la conjonctive qui s'en trouve soulevée) ou, à l'inverse, liées à un fonctionnement excessif de cette filtration.

Dans ce type de chirurgie on observe dans environ 70% des réussites chirurgicales (définies comme obtenant un abaissement suffisant de la PIO), outre la filtration sous-conjonctivale, une augmentation de l'écoulement uvéo-scléral, qui devient visible à l'examen par ultrasons. Cette augmentation étant l'un des mécanismes d'abaissement de la PIO il est intéressant de la provoquer chirurgicalement.

Il est connu du document WO 2010/088258 d'implanter, à partir de la chambre antérieure et jusque dans l'espace entre la sclère et le corps ciliaire, voire la choroide (espace suprachoroidal), un implant drainant dont la raideur est de nature à déformer les tissus qui l'entourent.

L'abaissement de la PIO obtenu par ces techniques est souvent insuffisant et temporaire. Il nécessite le plus souvent la reprise ou le maintien d'un ou de plusieurs traitements médicaux hypotenseurs.

Compte tenu de ce qui précède, la présente invention propose d'accroître et de rendre permanent l'effet hypotenseur de l'écoulement uvéo-scléral physiologique en interposant, entre la sclère et le corps ciliaire, un implant qui n'altère pas les structures anatomiques, avec ou sans intervention ajoutée, filtrante ou autre.

La présente invention a ainsi pour objet un implant ophtalmologique d'interposition permanente selon la revendication 1.

Le bord antérieur concave du corps de l'implant permet de positionner ledit bord antérieur au plus près du trabeculum et sur une circonférence, ce qui permet à l'implant d'exercer son effet d'écartement au meilleur endroit possible (la concavité du bord antérieur est adaptée généralement au rayon de la cornée). Cette configuration du bord antérieur concave permet de recueillir de manière permanente l'humeur aqueuse au plus près de la zone de l'écoulement uvéo-scléral. Un tel implant apporte ainsi un gain significatif dans l'augmentation de l'écoulement uvéo-scléral physiologique.

Le corps de l'implant peut présenter en projection dans un plan perpendiculaire à l'épaisseur un contour périphérique externe à géométrie convexe à l'exception de la portion du corps qui comporte le bord antérieur concave. La portion restante du corps en projection dans ce plan a une géométrie convexe, c'est-à-dire que chaque fois que l'on prend deux points A et B de cette portion restante le segment AB qui les relie est entièrement contenu dans ladite portion restante. Autrement dit, en dehors de la portion du corps qui inclut le bord antérieur concave (cette portion peut être délimitée, d'un côté, par le bord antérieur concave, du côté opposé, par une droite interne au corps et qui tangente le sommet de la courbure concave du bord et par deux bords opposés qui sont chacun adjacents au bord antérieur concave et à la droite tangente), en projection dans ledit plan la portion restante du corps ne comporte pas de creux ou de bosse, ni d'angle rentrant, ni de partie rentrante.

Par ailleurs, l'épaisseur du corps est unique au sens où le corps n'est pas formé de plusieurs parties comportant chacune une épaisseur différente et qui confèreraient au corps un profil à épaisseur variable (ex : profil en gradins, profil étagé ou en créneaux). Le corps est formé d'une seule partie en ce sens qu'il est homogène et ne comporte pas une forme complexe (avec des découpes, creux, retours...) avec des variations de géométrie locales, tant suivant l'épaisseur que perpendiculairement à l'épaisseur.

De manière générale le corps présente sur chacune de ses faces opposées deux à deux une forme plane ou générale incurvée à une seule courbure, c'est-à-dire qu'une telle forme avec des ondulations ou une forme en dents de scie est exclue.

Le corps ne présente généralement pas une forme avec des découpes, notamment suivant une vue en projection dans un plan perpendiculaire à l'épaisseur.

De manière générale, la forme générale du corps est simple comparée aux formes des corps d'implants de l'art antérieur.

De manière plus spécifique, le corps de l'implant selon l'invention est différent corps d'implant des documents WO 95/35078 (corps avec deux parties d'épaisseur différentes et avec des largeurs différentes perpendiculairement à l'épaisseur), US 4 521 210 (corps en forme de croix avec une branche plus allongée que les autres), US 2004/0015140 (corps en forme de spatule avec une portion antérieure de largeur réduite par rapport au reste du corps et avec des découpes latérales et une découpe postérieure axiale pour l'insertion d'un outil de mise en place de l'implant) et US 2004/0092856 (corps en forme de T avec les deux extrémités opposées de la barre du T d'épaisseur supérieure à celle de la partie restante du T).

Le document US-A-2004/254521 décrit un implant ophtalmologique d'interposition permanente entre la sclère et le tissu uvéal, comprenant un corps uvéocompatible formé d'une seule partie, le corps de l'implant comprenant deux bords opposés qui sont éloignés l'un de l'autre suivant une des deux dimensions perpendiculaires à l'épaisseur.

Le corps de l'implant est destiné à écarter la sclère et le tissu uvéal l'un de l'autre afin de s'interposer entre eux de manière permanente (création d'un espace permanent), créant ainsi une zone de moindre résistance à l'écoulement de l'humeur aqueuse. Un corps mince tel que défini ci-dessus procure un écartement suffisant des structures sus- et sous-jacentes (sclère et tissu uvéal) pour drainer efficacement l'écoulement uvéo-scléral physiologique.

On entend par corps uvéocompatible un corps dont le ou les matériaux constitutifs n'érodent ni ne dénaturent les structures oculaires avoisinantes, en l'occurrence les structures de l'uvée. La seule biocompatibilité du corps n'est pas suffisante pour que le corps puisse être implanté de façon permanente dans l'uvée, plus particulièrement entre la sclère et le tissu uvéal.

Selon d'autres caractéristiques possibles prises isolément ou en combinaison l'une avec l'autre :
- le corps est par exemple aminci au niveau du bord antérieur, par rapport au reste du corps, afin de pouvoir s'insérer sous l'éperon scléral, au plus près du trabeculum sans toutefois perturber la structure de l'éperon scléral ;
   - plus particulièrement, le bord antérieur concave est aminci sur une dimension qui est comprise entre 100 et 400µm, cette dimension étant prise suivant une direction qui s'étend entre les deux bords opposés du corps ; cette distance suivant laquelle le bord antérieur est aminci procure de bons résultats en termes d'efficacité de positionnement de l'implant au plus près du trabeculum sans toutefois perturber la structure de l'éperon scléral ; le bord postérieur opposé a par exemple une épaisseur identique au reste du corps de l'implant ; le corps conserve ainsi une épaisseur sensiblement uniforme (aux variations d'épaisseur locales possibles du corps) sur la partie qui ne comprend pas le bord aminci (lorsqu'un bord aminci est présent);
- les deux bords opposés du corps, à savoir le bord antérieur et le bord opposé postérieur, sont éloignés l'un de l'autre suivant la plus petite des deux dimensions perpendiculaires à l'épaisseur ;
- la courbure concave du bord antérieur présente un rayon de courbure compris entre 5 et 7 mm ; un tel rayon de courbure du bord antérieur permet de positionner ce bord de manière concentrique au limbe (transition entre cornée et sclère), assurant ainsi le recueil du maximum possible d'humeur aqueuse ;
- le corps de l'implant est élastiquement déformable de manière à pouvoir être plié sans induire de déformation permanente pour être manipulé avec un micro instrument, ou injecté avec un système d'injection ophtalmologique ; à l'état de repos, le corps non déformé est plan et, une fois implanté de manière permanente entre la sclère et le tissu uvéal, il est déformé dans un état dit déformé d'utilisation ;il épouse alors au plus près les courbures de la sclère et du corps ciliaire ;
- le corps de l'implant comporte, dans un état déformé dans lequel il est susceptible d'être utilisé comme implant ophtalmologique d'interposition entre la sclère et le tissu uvéal, une autre courbure concave dans une direction perpendiculaire à un plan défini par les deux dimensions de l'implant qui sont perpendiculaires à l'épaisseur ; cette deuxième courbure lui permet d'épouser au plus près la courbure du corps ciliaire en se moulant sur une portion de sphère de l'œil (corps ciliaire sous-jacent); l'implant comporte ainsi une double courbure ;
- le corps de l'implant est réalisé dans un matériau qui possède un module de Young compris entre 30 et 60 kg/cm2 ; cette caractéristique de rigidité/flexibilité permet au corps de se déformer élastiquement lors de sa mise en place dans l'œil et ensuite d'être maintenu dans un état déformé de manière permanente par les structures sus- et sous-jacentes de l'œil, en suivant les courbures naturelles de ces structures (une trop grande rigidité du corps induirait en effet des déformations de ces structures pouvant entrainer une altération de leur(s) fonction(s)) ; on notera que la rigidité définie ci-dessus est inférieure à celle de la sclère (voire très inférieure), ce qui permet à l'implant de tapisser la sclère sans la déformer ;
- le corps de l'implant n'est pas élastiquement déformable et comporte, de manière permanente, une deuxième courbure concave dans une direction perpendiculaire à un plan défini par les deux dimensions de l'implant qui sont perpendiculaires à l'épaisseur ;la deuxième courbure concave est choisie de manière à suivre les courbures naturelles des structures sus- et sous-jacentes de l'œil entre lequel le corps est implanté ;
- la deuxième courbure concave présente un rayon de courbure compris entre 10 et 15 mm ; ce rayon de courbure est particulièrement adapté pour que le corps de l'implant se moule sur la portion de sphère du corps ciliaire sous-jacent ;
- le corps de l'implant a, suivant une vue en trois dimensions, une forme générale de portion de calotte sphérique ;
- le corps de l'implant est apte à permettre un écoulement liquidien de l'humeur aqueuse à travers ledit corps et/ou le long de celui-ci; l'implant interposé entre le corps ciliaire et la sclère doit favoriser l'écoulement de l'humeur aqueuse recueilli au niveau du trabeculum vers la partie postérieure de l'œil, que ce soit le long du corps (de son bord antérieur à son bord postérieur opposé) et/ou à travers le corps ;
- le corps de l'implant possède deux grandes faces opposées écartées l'une de l'autre suivant l'épaisseur du corps ; les deux grandes faces opposées sont généralement parallèles entre elles et présentent la même courbure au repos et/ou lorsque l'implant est implanté (les deux grandes faces opposées peuvent en effet n'être courbées qu'après l'implantation) ;
   - les deux grandes faces opposées sont sensiblement planes ou courbées ;
   - les deux grandes faces opposées présentent chacune une ou plusieurs variations locales d'épaisseur comprise entre 10 et 20 µm ; ces variations locales d'épaisseur se produisent suivant une ou deux dimensions perpendiculaires à l'épaisseur qui sont petites devant la ou les dimensions du corps perpendiculaires à l'épaisseur (environ 10 fois plus petites que la ou les dimensions); on notera par ailleurs que la variation d'épaisseur possible est de l'ordre de 10% de l'épaisseur générale uniforme du corps ; ainsi une épaisseur sensiblement uniforme s'entend d'une épaisseur qui a une valeur générale ou nominale donnée et qui accepte localement une ou des variations d'épaisseur telles que définies ci-avant ;
- le corps de l'implant est percé d'orifices traversant son épaisseur ; ces orifices assurent le passage d'un écoulement d'humeur aqueuse à travers le corps ;
- le corps de l'implant comprend sur au moins une de ses deux grandes faces opposées un relief qui est apte à favoriser un écoulement de l'humeur aqueuse le long de ladite au moins une grande face ;
- le relief de ladite au moins une grande face du corps prend la forme de rainures aménagées sur ladite au moins une grande face ou d'une rugosité conférée à celle-ci ; ce relief peut par exemple prendre la forme de rainures ou rigoles agencées en surface sensiblement parallèlement à une direction qui s'étend du bord antérieur du corps au bord postérieur opposé de celui-ci ;
- le corps de l'implant comprend au moins un matériau qui est choisi parmi les matériaux suivants : le PTFE, le polysiloxane, les hydrogels acrylates hydrophiliques ou hydrophobiques ;
- le corps de l'implant possède, suivant une vue prise dans un plan contenant les projections des deux plus grandes dimensions dudit corps, des dimensions qui sont comprises entre des dimensions minimales de 2x 2mm et des dimensions maximales de 7x7mm ; le respect de ces dimensions permet à l'implant de garantir l'action d'écoulement uvéo-scléral souhaitable : les dimensions minimales garantissent d'assurer un effet d'interposition suffisant pour diminuer la résistance à l'écoulement et les dimensions maximales permettent de ne pas perturber la dynamique de l'humeur aqueuse par un écoulement trop important (une telle perturbation risquerait d'entrainer une hypotonie oculaire);
- l'épaisseur du corps de l'implant est comprise entre 50 et 400µm ; une telle épaisseur offre un écartement efficace ;
- le corps de l'implant possède un volume compris entre 0,8 et 8 mm3 ; un tel volume permet d'accroître l'écoulement uvéo-scléral sans excès ;
- le corps de l'implant présente des propriétés de relargage d'une ou de plusieurs substances ;
   - le corps a une surface externe qui ne présente pas d'arêtes vives ni d'angles vifs ; la surface externe présente ainsi uniquement des angles ou bords arrondis (quelle que soit l'orientation spatiale, à savoir en projection dans un plan perpendiculaire à l'épaisseur ou dans un plan incluant la direction suivant laquelle s'étend l'épaisseur) afin de faciliter la mise en place de l'implant et d'enlever tout caractère potentiellement traumatisant pour les tissus de voisinage ;
- le corps a, suivant une vue en projection dans un plan perpendiculaire à l'épaisseur, quatre bords ou côtés délimitant le contour extérieur dudit corps : le bord antérieur et le bord opposé postérieur et deux bords latéraux adjacents auxdits bords antérieur et postérieur et qui relient ces derniers entre eux (polygone à quatre côtés);
- les deux bords latéraux convergent l'un vers l'autre en allant du bord postérieur au bord antérieur (bord antérieur plus court que le bord postérieur) ou sont parallèles entre eux.
- le corps a, suivant une vue en projection dans un plan perpendiculaire à l'épaisseur, une forme générale de segment annulaire.

D'autres caractéristiques et avantages apparaitront au cours de la description qui va suivre, donnée uniquement à titre d'exemple non limitatif et faite en référence aux dessins annexés, sur lesquels :
- la figure 1 est une vue générale très schématique montrant en vue de face plusieurs formes de réalisation possibles d'implants selon l'invention positionnés autour de la cornée d'un œil ;
- les figures 2a à 2c illustrent différentes formes de réalisation possibles d'implants selon l'invention ;
- les figures 3, 4 et 5 illustrent schématiquement chacune une forme de réalisation possible d'une configuration d'un implant qui favorise l'écoulement de l'humeur aqueuse à travers (fig.3), le long (fig.4), à travers et le long (fig.5) du corps d'un implant selon l'invention ;
- les figures 6 et 7 illustrent de manière très schématique la mise en place d'un implant suivant deux méthodes différentes ;
- la figure 8 est une vue de mise en situation schématisée dans un œil humain d'une forme de réalisation possible d'un implant selon l'invention interposé entre la sclère et le corps ciliaire ;
- la figure 9 est une vue plus détaillée et agrandie de la structure de l'angle iridocornéen sans l'implant de la figure 8 ;
- les figures 10a et 10b sont des vues comparatives montrant l'amélioration de l'écoulement d'humeur aqueuse en présence d'un implant conforme à un mode de réalisation de l'invention entre la sclère et l'uvée.

L'invention concerne un implant ophtalmologique qui est destiné à être implanté de manière permanente entre la sclère et le tissu uvéal et donc à être interposé de manière permanente entre ces derniers.

L'implant selon l'invention comprend un corps mince afin que, une fois interposé entre la sclère et le tissu uvéal, il ne déforme pas les tissus sus- et sous-jacents de manière inacceptable. Une déformation acceptable des tissus est une déformation qui n'altère pas la ou les fonctions de l'un et/ou de l'autre de ces tissus.

Le corps de l'implant comporte trois dimensions dans l'espace : une épaisseur, une longueur et une largeur qui sont perpendiculaires à l'épaisseur (ou deux dimensions identiques si la longueur et la largeur sont égales). On entend par « corps mince » un corps dont l'épaisseur est au moins inférieure à 10 fois la plus petite des deux autres dimensions du corps, à savoir la largeur ou n'importe laquelle des deux autres dimensions en cas d'égalité de ces dernières. Dans un exemple de réalisation, l'épaisseur est inférieure à 13 fois la largeur du corps. A titre d'exemple, pour un corps avec des dimensions (largeur et longueur) de 4x5 mm, son épaisseur est de 0,3 mm.

Le corps de l'implant comprend deux bords opposés qui sont éloignés l'un de l'autre suivant une des deux dimensions perpendiculaires à l'épaisseur, par exemple la largeur (plus petite des deux dimensions).

L'un des deux bords distants est appelé bord antérieur et présente, en projection dans un plan perpendiculaire à l'épaisseur, une courbure concave tournée vers l'extérieur du corps.

La courbure concave du bord antérieur permet de positionner ce bord de l'implant au plus près du trabeculum, c'est-à-dire de la zone de l'écoulement uvéo-scléral, afin de recueillir la plus grande quantité possible d'humeur aqueuse.

Le rayon de courbure du bord concave est, par exemple, compris entre 5 et 7 mm, ce qui permet de positionner ce bord de manière concentrique au limbe, et donc au plus près du trabeculum. La concentricité est définie par rapport au centre de la cornée (centre du cercle) qui n'est pas toujours aligné avec le centre de la pupille. Le rayon de courbure est par exemple égal à 5,5mm.

L'autre bord opposé est appelé bord postérieur et il n'est pas nécessairement courbé (convexité tournée vers l'extérieur) dans un plan perpendiculaire à l'épaisseur. Il peut en effet être rectiligne, voire adopter une forme différente.

Le corps de l'implant comprend au moins un matériau qui est connu pour ses propriétés d'uvéocompatibilité et le corps est ainsi uvéocompatible. De telles propriétés procurent au corps une très faible adhérence aux tissus oculaires. En d'autres termes, ledit au moins un matériau n'est pas susceptible d'altérer les structures sus-jacentes et sous-jacentes par suite de la mise en contact du corps avec ces structures et de leurs mouvements répétés au cours du temps.

De manière générale, le corps de l'implant peut présenter en projection dans un plan perpendiculaire à l'épaisseur un contour périphérique externe à géométrie convexe à l'exception de la portion du corps qui comporte le bord antérieur concave. La portion restante du corps en projection dans ce plan a une géométrie convexe, c'est-à-dire que chaque fois que l'on prend deux points A et B de cette portion restante le segment AB qui les relie est entièrement contenu dans ladite portion restante. Autrement dit, en dehors de la portion du corps qui inclut le bord antérieur concave (cette portion peut être délimitée, d'un côté, par le bord antérieur concave, du côté opposé, par une droite fictive interne au corps et qui tangente le sommet de la courbure concave du bord et par deux bords opposés qui sont chacun adjacents au bord antérieur concave et à la droite tangente), en projection dans ledit plan la portion restante du corps ne comporte pas de creux ou de bosse, ni d'angle rentrant, ni de partie rentrante.

Par ailleurs, l'épaisseur du corps est unique au sens où le corps n'est pas formé de plusieurs parties comportant chacune une épaisseur différente et qui confèreraient au corps un profil à épaisseur variable (ex : profil en gradins, profil étagé ou en créneaux).

De manière générale le corps présente sur chacune de ses faces opposées deux à deux une forme plane ou générale incurvée à une seule courbure, c'est-à-dire qu'une telle forme avec des ondulations ou une forme en dents de scie est exclue.

Le corps ne présente généralement pas une forme avec des découpes, notamment suivant une vue en projection dans un plan perpendiculaire à l'épaisseur.

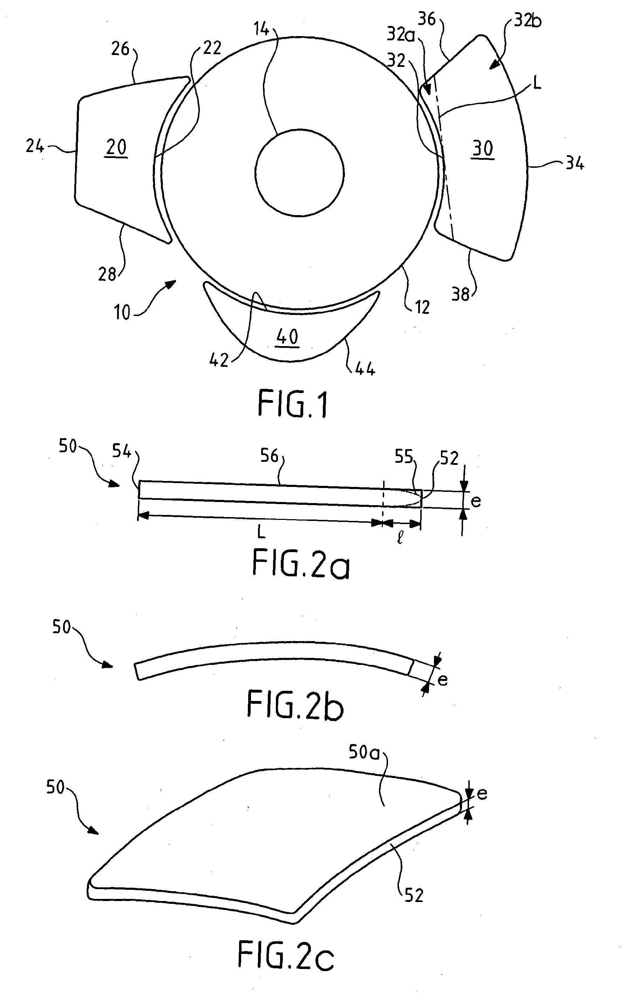

Comme représenté à la figure 1, un œil 10 est représenté en vue de face de manière très schématique par la cornée 12 et la pupille 14 au centre.

Trois formes possibles de réalisation d'un implant ophtalmologique selon l'invention sont représentées en position implantée autour de la cornée suivant une vue en projection dans un plan perpendiculaire à l'épaisseur du corps (plan de la figure 1).

Ces trois formes possibles d'implants référencées 20, 30 et 40 ont chacune en commun un bord antérieur concave 22, 32, 42 dont la concavité, c'est-à-dire le rayon de courbure, est ajustée pour que ledit bord puisse être disposé de manière concentrique au limbe, à savoir au plus près du trabeculum.

Ces trois implants ont chacun un bord postérieur opposé 24, 34, 44 différent du bord antérieur et des bords latéraux différents d'un implant à l'autre.
- Le bord postérieur 24 de l'implant 20 est rectiligne et le corps comporte deux bords latéraux 26, 28 adjacents aux bords antérieur et postérieur et reliant ces derniers entre eux; les bords latéraux 26, 28 divergent l'un par rapport à l'autre en allant du bord postérieur au bord antérieur dans la mesure où le bord antérieur 22 est plus long que le bord postérieur 24 ; les bords latéraux 26, 28 sont symétriques l'un par rapport à l'autre bien que, selon une variante non représentée, ils puissent être asymétriques l'un par rapport à l'autre.

Le bord postérieur 34 de l'implant 30 est convexe (convexité tournée vers l'extérieur du corps ; alternativement, le bord postérieur peut être rectiligne) et le rayon de courbure peut être ou non identique à celui du bord concave 32.Le corps comporte deux bords latéraux 36, 38 adjacents aux bords antérieur et postérieur et reliant ces derniers entre eux; les bords latéraux 36, 38 convergent l'un vers l'autre en allant du bord postérieur au bord antérieur dans la mesure où le bord antérieur 32 est plus court que le bord postérieur 34. De manière générale, le corps de l'implant 30 a une forme en vue de dessus (perpendiculaire à son épaisseur) qui ressemble à un trapèze dont la base est concave et le sommet convexe ou à un segment ou portion annulaire qui s'étend suivant un secteur angulaire donné.

Le corps de l'implant 30 présente ici, en projection dans un plan perpendiculaire à l'épaisseur, un contour périphérique externe à géométrie convexe à l'exception de la portion 32a du corps qui comporte le bord antérieur concave 32. La portion restante 32b du corps en projection dans ce plan a une géométrie convexe, c'est-à-dire que chaque fois que l'on prend deux points A et B de cette portion restante le segment AB qui les relie est entièrement contenu dans ladite portion restante. Cette portion est délimitée, d'un côté, par le bord antérieur concave 32, du côté opposé, par une droite ou axe fictif L interne au corps et qui tangente le sommet de la courbure concave du bord (du côté de la convexité de la courbure) et par deux bords opposés qui sont chacun adjacents au bord antérieur concave et à la droite ou axe tangent L. Autrement dit, en dehors de la portion 32a du corps qui inclut le bord antérieur concave, en projection dans ledit plan la portion restante du corps ne comporte pas de creux ni de bosse, ni d'angle rentrant, ni de partie rentrante. On notera que d'autres géométries convexes peuvent être envisagées pour el corps.

Selon une variante non représentée les bords latéraux peuvent être parallèles entre eux. Selon une autre variante non représentée, la forme générale du corps en vue dessus peut être celle d'un rectangle à l'exception du bord antérieur concave, le bord postérieur pouvant être rectiligne ou convexe.

Le bord postérieur 44 convexe de l'implant 40 (convexité tournée vers l'extérieur du corps) rejoint directement le bord antérieur 42, il n'y a pas de bords latéraux adjacents ; le corps a ainsi une forme générale de croissant de lune en vue de dessus (fig. 1) et présente ainsi entre les deux bords antérieur 42 et postérieur 44 une dimension relativement faible par rapport aux implants 20 et 30 où les deux bords sont davantage écartés l'un de l'autre. La forme de l'implant 40 facilite sa mise en place à travers une incision de plus petite taille que pour les implants 20 et 30, tout en prolongeant l'effet d'écartement vers l'arrière (bord postérieur).

Dans certaines configurations d'implants il est envisagé de maximiser la longueur du bord antérieur, la configuration et/ou la longueur du bord postérieur étant, quant à elles, plus libres.

Il convient de noter que, de manière générale, les bords latéraux de l'implant (quand ils existent, ce qui n'est pas le cas avec l'implant 40 de la figure 1) peuvent être agencés de manière radiale comme pour l'implant 30 (bords radiaux relativement au centre du cercle fictif par rapport auquel le bord concave de l'implant est positionné ; les bords sont ainsi convergents vers le centre du cercle) ou évasée comme pour l'implant 20.

De manière générale (quelle que soit la forme de l'implant), le bord postérieur doit être suffisamment éloigné du bord antérieur afin de procurer un effet d'écartement efficace. En pratique, le bord postérieur est éloigné du bord antérieur d'une distance d'au moins 3 mm.

De manière générale (quelle que soit la forme de l'implant), la longueur du bord postérieur ne dépasse pas de plus de 10% la longueur du bord antérieur.

Toutefois, selon des variantes non représentées, d'autres implants présentant des formes et/ou des dimensions différentes peuvent également convenir : ainsi, un implant peut s'étendre sur une plus grande circonférence (ou secteur angulaire) autour de la cornée 12 que ce qui est représenté sur la figure 1.

On notera que, de manière générale (quelle que soit la forme de l'implant) la longueur du bord antérieur (portion de circonférence de cercle) est par exemple comprise entre 3 et 7 mm. Au-delà de cette longueur, l'implant s'avère plus difficile à mettre en place (taille de l'incision plus grande...).

L'effet procuré par un implant au sens général de l'invention (augmentation de l'écoulement uvéo-scléral physiologique) peut être obtenu avec un ou plusieurs implants disposés autour de la cornée, l'un contre l'autre ou bien éloignés l'un de l'autre.

De manière générale (quelle que soit la forme de l'implant), les différents bords ou faces ou tranches ou flancs délimitant la surface extérieure de l'implant et qui sont adjacents les uns aux autres sont reliés entre eux par des arêtes, angles ou coins qui sont arrondis. En d'autres termes, le corps de l'implant ne comporte pas d'arêtes vives.

Comme indiqué plus haut, le corps de l'implant a également une épaisseur uniforme et, suivant une vue en trois dimensions, il présente, au repos, une forme plane (le corps ne présente pas de deuxième courbure dans une direction perpendiculaire à un plan défini par les deux dimensions du corps en vue de dessus comme celui de la figure 1) ou incurvée (au moins une deuxième courbure présente suivant cette direction perpendiculaire au plan des deux autres dimensions).

La figure 2a illustre un implant 50 vu suivant son épaisseur notée « e », c'est-à-dire dans une direction qui est comprise dans le plan de la figure 1. Un tel implant a un bord antérieur concave dont la concavité n'est pas visible ici.

Cet implant comporte donc la courbure concave du bord antérieur dans un plan défini par les deux autres dimensions du corps (en dehors de son épaisseur) mais, au repos (état non déformé), il est plan suivant une direction perpendiculaire à ce plan (absence de deuxième courbure).

Le corps de cet implant est réalisé dans un matériau élastiquement déformable de manière à pouvoir être plié sans induire de déformation permanente pour être manipulé avec un micro instrument, ou injecté avec un système d'injection ophtalmologique. Lorsqu'un tel corps n'est plus soumis à l'effort de pliage, il reprend sa position d'origine non déformée (repos).

Les figures 2b et 2c illustrent le corps de la figure 2a dans un état déformé (fig.2b : vue de profil suivant l'épaisseur ; fig.2c : vue en perspective de dessus), par exemple lorsqu'il a été implanté entre la sclère et le tissu uvéal. Cet état déformé peut également être obtenu lorsqu'un effort externe de pliage est imposé au corps par un outil tel qu'un micro instrument ou un système d'injection ophtalmologique d'injection.

Le corps tel que représenté à la figure 2c épouse une portion de surface sphérique et a une forme générale de portion de calotte sphérique. La forme du corps est donc particulièrement adaptée pour épouser une portion de la surface sphérique du corps ciliaire sous-jacent à l'implant. Ce corps comprend le bord antérieur concave 52.

La forme adoptée par le corps dans cet état déformé montre que celui-ci comporte (en plus de la courbure concave du bord antérieur) une double courbure suivant une direction perpendiculaire au plan défini par les deux autres dimensions du corps (longueur et largeur) illustrées sur la figure 1. Sur la figure 2b, une seule de ces autres courbures (en plus de la courbure concave du bord antérieur) est représentée.

Ainsi déformé, le corps comporte deux grandes faces opposées écartées l'une de l'autre suivant l'épaisseur : une face supérieure 50a convexe qui est doublement bombée et une face inférieure opposée non représentée concave qui est également doublement bombée et qui est destinée à épouser une portion de surface sphérique. Cette double courbure permet à l'implant de bien répartir l'effet d'écartement entre les tissus.

La face supérieure 50a convexe est destinée à être en vis-à-vis de la sclère, tandis que la face inférieure concave est destinée à être en vis-à-vis du tissu uvéal.

On notera que chaque courbure de la double courbure précitée possède un même rayon de courbure compris entre 10 et 15 mm et, par exemple, qui est égal à 11 mm.

Le corps de l'implant est réalisé dans un matériau qui possède un module de Young compris entre 30 et 60 kg/cm2. Un tel module procure au corps une caractéristique de flexibilité lui permettant de se déformer élastiquement lors de sa mise en place dans l'œil et ensuite d'être maintenu dans un état déformé de manière permanente par les structures sus- et sous-jacentes de l'œil, en suivant les courbures naturelles de ces structures. Un tel module permet de ne pas occasionner des déformations dans l'une et/ou l'autre de ces structures qui seraient de nature à altérer leur(s) fonction(s).

Dans un exemple de réalisation, le module de Young est égal à 40 kg/cm2 pour un corps élastiquement déformable réalisé dans un matériau acrylique hydrophile qui est par exemple hydrophile à 25%.

On notera que tout ce qui vient d'être dit à propos du mode de réalisation des figures 2a-c s'applique également à un implant dont le corps a une forme générale différente.

Le contour du corps, vu dans en projection un plan défini par les deux plus grandes dimensions comme sur la figure 1 (autrement dit un plan perpendiculaire à l'épaisseur), peut différer de celui illustré sur la figure 2c, à Iexception du bord antérieur 52 qui reste concave.

Par ailleurs, le bord antérieur peut ne pas être aminci comme sur la figure 2a.

Bien que sur les figures 2a-c les bords, flancs ou tranches du corps qui s'étendent suivant l'épaisseur du corps semblent avoir des arêtes vives entre les faces adjacentes, cela n'est pas le cas. Toutes ces arêtes sont arrondies quelle que soit l'orientation géométrique afin de faciliter la mise en place de l'implant.

Selon une variante de réalisation, le corps de l'implant est réalisé dans un matériau qui n'est pas élastiquement déformable et qui présente, de manière permanente, au moins une deuxième courbure suivant la direction perpendiculaire au plan des deux autres dimensions (largeur et longueur sur la figure 1), comme illustré sur la figure 2b.

Cette autre courbure est donc présente lorsque le corps est au repos.

Une fois implanté dans sa position finale d'interposition permanente entre la sclère et le tissu uvéal, le corps ne se déforme pas plus et conserve cette autre courbure permanente.

On notera que selon cette variante, le corps peut adopter la forme générale de la figure 2c ou une forme générale différente comme expliqué ci-dessus pour les variantes appliquées aux figures 2a-c.

Un exemple de matériau non élastiquement déformable pouvant être utilisé est le polysiloxane.

Le corps de l'implant possède, suivant une vue prise dans un plan contenant les projections des deux plus grandes dimensions dudit corps, à savoir le plan de la figure 1, des dimensions qui sont généralement comprises entre des dimensions minimales de 2x 2mm et des dimensions maximales de 7x7mm. De telles dimensions permettent à l'implant d'assurer l'action nécessaire pour augmenter l'écoulement uvéo-scléral physiologique qui, en l'absence de l'invention, est d'environ 10% dans un œil humain. Ceci permet de diminuer la pression intra-oculaire.

Ces dimensions sont suffisantes pour que l'effet d'écartement des tissus soit efficace et réduise ainsi la résistance à l'écoulement d'humeur aqueuse et elles ne sont pas trop élevées afin de ne pas perturber la dynamique de cet écoulement. L'implant tel qu'illustré sur la figure 2c permet ainsi d'obtenir un écoulement uvéo-scléral physiologique d'environ 30 à 40%, ce qui permet d'abaisser de manière considérable la pression intra-oculaire (PIO).

Sur les figures 2a et 2b l'épaisseur du corps a été représentée de manière identique sur tout le corps.

Toutefois, le bord antérieur concave peut être aminci.

Dans l'exemple de la figure 2a (mais cela s'appliquerait aussi aux figures 2b et 2c) le bord antérieur concave 52 a été représenté de manière amincie sur une distance ou dimension « I » qui est comprise entre 100 et 400µm. Cette dimension est prise suivant une direction qui s'étend entre les deux bords opposés antérieur 52 et postérieur 54 du corps. La portion amincie est notée 55. La portion restante notée 57 du corps de longueur « L » possède par exemple une épaisseur identique constante jusqu'au bord postérieur inclus.

Toutefois, selon une variante non représentée, l'épaisseur de la portion restante du corps de longueur « L » a une valeur non nécessairement uniforme sur tout ou partie de cette longueur.

De manière générale (quelle que soit la forme de l'implant), l'épaisseur du bord postérieur est au moins égale à celle du bord antérieur.

On notera que l'épaisseur uniforme du corps de l'implant est de manière générale comprise entre 50 et 400µm, assurant ainsi un écartement suffisant et efficace des tissus. En dessous de 50 µm, l'effet d'écartement n'existe pas ou très peu. Au dessus de 400µm, un risque d'hypotonie est envisageable.

La longueur l d'amincissement du bord antérieur (portion 55) procure de bons résultats en termes d'efficacité de positionnement de l'implant au plus près du trabeculum, sans toutefois perturber la structure de l'éperon scléral et donc son insertion sur le corps ciliaire.

Le corps de l'implant possède un volume compris entre 0,8 et 8 mm3, ce qui lui permet d'assurer l'écoulement uvéo-scléral souhaité sans occasionner des problèmes de déformation inacceptables des tissus sus- et sous-jacents et une perturbation de la dynamique de l'humeur aqueuse.

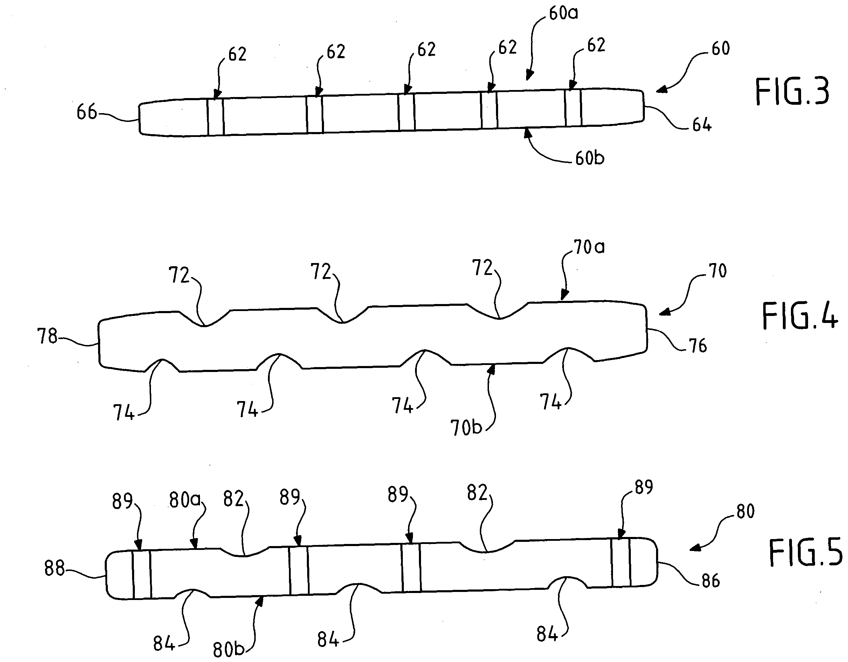

Les figures 3 à 5 illustrent un autre aspect d'un implant selon un autre mode de réalisation de l'invention et représentent trois implants 60, 70 et 80 vus de profil, c'est-à-dire suivant leur épaisseur. Sur ces figures, aucune courbure n'a été représentée telle que celle(s) illustrée(s) sur les figures 2b et 2c par souci de simplification. Toutefois, la description qui suit s'applique à des implants dont le corps est élastiquement déformable ou non, avec simple ou double courbure suivant la direction de l'épaisseur et quelles que soient la forme et les dimensions de l'implant. Sur les figures 3 à 5, les dimensions ont été volontairement exagérées pour des besoins de compréhension.

Selon cet aspect, le corps de l'implant est apte à permettre un écoulement liquidien de l'humeur aqueuse à travers ledit corps (figure 3) ou le long de celui-ci (figure 4), voire à travers et également le long du corps (figure 5). En effet, l'implant qui est interposé entre le corps ciliaire et la sclère doit favoriser l'écoulement/drainage de l'humeur aqueuse recueilli au niveau du trabeculum vers la partie postérieure de l'œil. Cet écoulement peut être favorisé le long du corps (de son bord antérieur à son bord postérieur opposé) et/ou à travers le corps.

Comme illustré sur la figure 3, le corps de l'implant 60 est percé d'orifices 62 traversant son épaisseur. Ces orifices 62 assurent le passage d'un écoulement d'humeur aqueuse à travers le corps, d'une des deux grandes faces opposées 60a à l'autre grande face opposée 60b. Les orifices sont ici représentés alignés suivant une direction s'étendant du bord antérieur concave 64 au bord postérieur opposé 66 dans un plan de coupe. Le corps est également percé d'une pluralité d'autres orifices non représentés qui sont situés dans d'autres plans devant et derrière le plan de coupe de la figure3.

Toutefois, les orifices traversant le corps ne sont pas nécessairement alignés comme illustré sur la figure 3. A titre d'exemple, chaque orifice a un diamètre de 50µm et l'épaisseur du corps est de 200µm.

Comme illustré sur la figure 4, le corps de l'implant 70 comprend sur au moins une de ses deux grandes faces opposées 70a, 70b un relief qui est apte à favoriser un écoulement de l'humeur aqueuse le long de ladite au moins une grande face.

Dans l'exemple représenté les deux grandes faces 70a et 70b sont pourvues d'un tel relief qui, comme on le voit, n'est pas nécessairement identique d'une face à l'autre. Toutefois, le relief peut être le même d'une face à l'autre.

Le relief prend par exemple la forme de rainures ou rigoles 72, 74 aménagées respectivement sur les faces 70a, 70b, en surface de celles-ci. Ces rainures ou rigoles 72, 74 sont préférentiellement agencées sensiblement parallèlement à une direction qui s'étend du bord antérieur concave du corps au bord postérieur opposé de celui-ci.

Sur la figure 4, cette direction est perpendiculaire au plan de la figure, les bords antérieur et postérieur n'ayant pas été représentés. Seuls les bords adjacents opposés 76, 78 sont représentés.

Les rainures ou rigoles 72 ne sont pas disposées en vis-à-vis des rainures ou rigoles 74 afin de ne pas fragiliser la constitution du corps en réduisant son épaisseur localement à chaque endroit où les deux rainures ou rigoles seraient en vis-à-vis l'une de l'autre.

On notera que le corps de l'implant peut combiner les orifices traversants et les rainures : les orifices sont par exemple placés au fond des rainures ou entre deux rainures adjacentes disposées sur une même face.

L'implant 80 de la figure 5 comprend, aménagées sur la grande face 80a, des rainures ou rigoles 82 et, de manière décalée, aménagées sur la grande face opposée 80b, des rainures ou rigoles 84. Des orifices traversants 89 sont pratiqués dans l'épaisseur du corps, à différents emplacements, entre les rainures des deux faces et/ou de part et d'autre des rainures. Le nombre, les dimensions et la localisation des rainures et des orifices peut varier.

Comme sur la figure 4, les rainures des deux faces opposées sont disposées en quinconce, pour les mêmes raisons.

Sur la figure 5, les bords antérieur et postérieur n'ont pas été représentés, comme sur la figure 4, seuls les bords adjacents opposés 86, 88 ayant été représentés.

Selon une variante non représentée, le relief peut prendre la forme d'un rugosité ou texture conférée à l'une et/ou à l'autre d'une des deux grandes faces opposées du corps par une méthode connue.

De manière plus générale et non représentée, un implant selon une variante de l'implant de la figure 5 intègre à la fois des orifices traversants et un relief qui est différent des rainures ou rigoles précitées. Ce relief pouvant être le même ou varier d'une face à l'autre.

On notera que le relief décrit ci-dessus en relation avec les figures 4 et 5 peut s'apparenter à des variations locales d'épaisseur comprises entre 10 et 20 µm sur chacune (ou sur l'une seulement) des deux grandes faces opposées du corps. Ces variations locales d'épaisseur se produisent suivant une ou deux dimensions perpendiculaires à l'épaisseur qui sont petites devant la ou les dimensions du corps perpendiculaires à l'épaisseur (environ 10 fois plus petites que la ou les dimensions). On notera que la variation d'épaisseur est de l'ordre de 10% de l'épaisseur générale uniforme du corps. Ainsi, le corps illustré sur les figures 4 et 5 a une épaisseur sensiblement uniforme, c'est-à-dire une épaisseur qui a une valeur générale ou nominale donnée et qui accepte localement des variations d'épaisseur comprises entre 10 et 20 µm.

De manière générale, le ou les matériaux constitutifs du corps de l'implant sont choisis parmi les matériaux suivants : le PTFE, le polysiloxane, les hydrogels acrylates hydrophiliques ou hydrophobiques.

Le corps de l'implant présente des propriétés de relargage d'une ou de plusieurs substances. De telles substances sont par exemple des substances anti-infectieuses et/ou anti-inflammatoires. Il peut ainsi s'agir de substances antibiotiques et/ou de substances cortisoniques ou anti-cortisoniques.

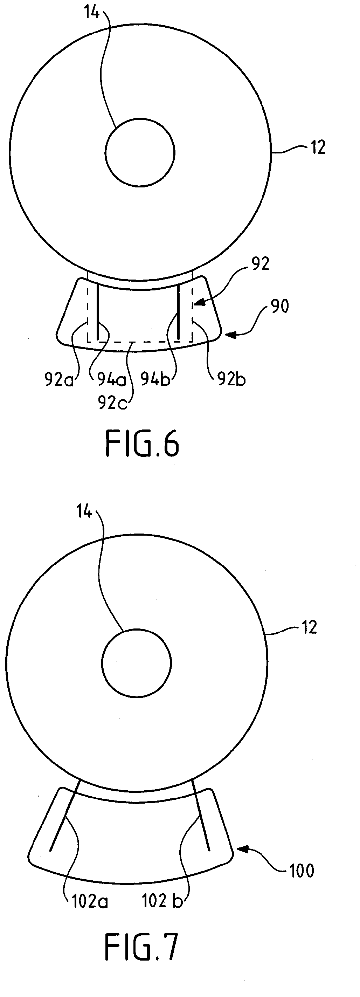

La figure 6 illustre la mise en place d'un implant 90 en utilisant une première méthode d'implantation selon un mode de réalisation de l'invention. Cette première méthode est utilisée en complément d'une intervention chirurgicale conventionnelle anti-glaucomateuse ou en complément de toute intervention intra oculaire lorsqu'un abaissement de la pression intra oculaire est souhaitable. La trabeculectomie et la sclérectomie nécessitent de découper un ou plusieurs volet(s) scléral(aux) de l'œil qui seront soulevés afin de poursuivre l'intervention. Un volet scléral 92 (illustré en pointillés sur la figure 6) est obtenu en incisant la sclère en un ou deux plans et à une profondeur variable, sur trois côtés : deux incisions 92a, 92b sensiblement parallèles entre elles qui s'étendent à partir de la cornée 12 et en éloignement de celle-ci et une troisième incision 92c perpendiculaire aux deux autres incisions et à distance de la cornée. La découpe suivant trois incisions ainsi réalisée forme ce que l'on appelle un ou plusieurs volet(s) scléral(aux). Après soulèvement du ou des volet(s) scléral(aux), deux incisions (94a, 94b sur la figure 6) sont pratiquées jusqu'au corps ciliaire, à l'intérieur du volet 92, afin de pouvoir glisser l'implant entre le plan scléral profond et le corps ciliaire. A titre d'exemple, les incisions sont espacées l'une de l'autre d'au moins 2 mm.

Selon une variante non représentée, une seule incision du plan scléral profond est réalisée pour atteindre le même but.

La méthode d'implantation utilisée comprend ensuite une étape d'introduction d'une substance visco-élastique, par exemple de type acide hyaluronique, à travers au moins une des incisions réalisées, entre la sclère et le corps ciliaire afin de séparer ces deux tissus précédemment accolés. Ceci permettra la mise en place de l'implant sans traumatisme pour les structures sus- et sous-jacentes.

Cette étape est mise en œuvre par l'intermédiaire d'un instrument d'injection telle qu'une canule d'injection ayant un diamètre de l'ordre de 20 à 30g.

Une faible quantité de substance est injectée, par exemple 0,05 mm3. La méthode d'implantation comprend également une étape d'introduction d'un instrument tel qu'une pince à bords mousses à travers une des deux incisions 94a, 94b qui s'étendent en profondeur jusqu'au corps ciliaire. La pince ressort par la deuxième incision et saisit l'implant pour venir le placer entre la sclère et le corps ciliaire.

Au cours d'une étape suivante, avec un instrument micro chirurgical tel qu'une spatule à bords mousses on s'assure de la position de l'implant 90 au plus près du trabeculum (de manière concentrique au limbe), comme exposé supra, afin de recueillir le maximum d'humeur aqueuse.

Un autre type d'instrument ou de dispositif permettant la mise en place, le déploiement de l'implant et son positionnement dans l'espace situé entre la sclère et le corps ciliaire (espace supra-choroidien) peut être utilisé, tel qu'un injecteur.

Au cours d'une autre étape, le ou les volet(s) scléral(aux) sont rabattus et, suturés ou non.

La figure 7 illustre la mise en place d'un implant 100 en utilisant une deuxième méthode d'implantation selon un mode de réalisation de l'invention.

Cette méthode est très similaire à la première méthode excepté le fait que la deuxième méthode ne vient pas en complément d'une intervention conventionnelle mais constitue une intervention en soi.

Selon cette méthode :
- deux incisions 102a, 102b radiales par rapport à la cornée ou parallèles entre elles (comme les incisions 94a, 94b de la figure 6) sont pratiquées à partir du limbe (zone de transition entre la cornée et la sclère ), sur une longueur allant par exemple de 1 à 4 mm, et poursuivies jusqu'au corps ciliaire,
- une substance visco-élastique, par exemple de type acide hyaluronique, est injectée à travers l'une des deux incisions,
- la sclère est soulevée afin de permettre l'insertion et la mise en place de l'implant 100.

Ces étapes sont identiques à celles décrites précédemment pour la première méthode.

L'étape finale de suture des incisions est toujours optionnelle.

Selon une variante de réalisation non représentée, une seule incision est pratiquée au cours de cette deuxième méthode et suffit pour venir installer en position d'interposition un implant entre la sclère et le corps ciliaire.

On notera que les implants 90 et 100 représentés sur les figures 6 et 7 peuvent être l'un quelconque des implants décrits ci-dessus. Les méthodes de mise en place d'implants décrites ci-dessus s'appliquent à tout implant selon l'invention et notamment à un implant ophtalmologique d'interposition permanente entre la sclère et le tissu uvéal qui comprend un corps mince uvéocompatible formé d'une seule partie, le corps ayant une seule épaisseur e sensiblement uniforme qui est au moins inférieure à 10 fois la plus petite des deux autres dimensions du corps, le corps de l'implant comprenant deux bords opposés qui sont éloignés l'un de l'autre suivant une des deux dimensions perpendiculaires à l'épaisseur, l'un des bords appelé bord antérieur présentant, en projection dans un plan perpendiculaire à l'épaisseur, une courbure concave tournée vers l'extérieur du corps . L'implant peut en outre comporter l'une quelconque (ou plusieurs, voire toutes) des caractéristiques présentées dans la description générale ainsi que dans les différents modes de réalisation et variantes.

La deuxième méthode décrite s'applique à la mise en place de plusieurs implants selon l'invention. De manière générale, au moins une incision différente (voire deux dans l'exemple de la figure 7) est à pratiquer pour la mise en place de chaque implant différent.

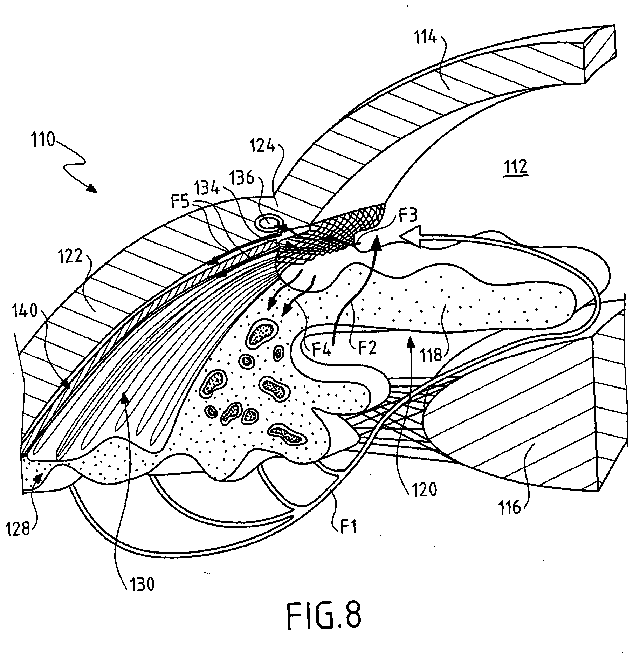

La figure 8 représente, en coupe, un implant selon un mode de réalisation de l'invention qui a été mis en place suivant l'une des méthodes décrites ci-dessus.

Cette coupe d'une partie d'un œil 110 représente la chambre antérieure 112 qui est disposée entre la cornée 114 et le cristallin 116 délimité à sa partie périphérique par l'iris 118.

Derrière l'iris 118 est disposée la chambre postérieure 120.

La sclère 122 est raccordée à la périphérie de la cornée 114 via le limbe 124 (zone de changement du rayon de courbure entre la sclère et la cornée). La sclère 122 recouvre le corps ciliaire 128 qui est raccordé à l'iris 118 et qui comprend le muscle ciliaire 130 sur lequel s'appuie la sclère 122.

Le trabeculum 134 disposé entre la cornée et l'iris fait office de filtre et est traversé par l'humeur aqueuse qui circule dans la chambre antérieure 112.

Le canal de Schlemm 136 est situé entre la sclère et la cornée en arrière du trabeculum 134.

Les différentes flèches F1, F2, F3 et F4 illustrent les chemins ou trajets empruntés par l'humeur aqueuse :
- F1 représente le chemin ou flux conventionnel emprunté par l'humeur aqueuse entrant dans la chambre antérieure 112 ;
- F2 représente le chemin ou flux de diffusion emprunté par l'humeur aqueuse pour entrer dans la chambre antérieure 112 ;
- F3 représente le chemin ou flux conventionnel emprunté par l'humeur aqueuse sortant de la chambre antérieure 112 à travers le trabeculum 134 et se dirigeant vers le canal de Schlemm 136 ;
- F4 représente l'écoulement physiologique uvéo-scléral conventionnel de l'humeur aqueuse sortant de la chambre antérieure 112.

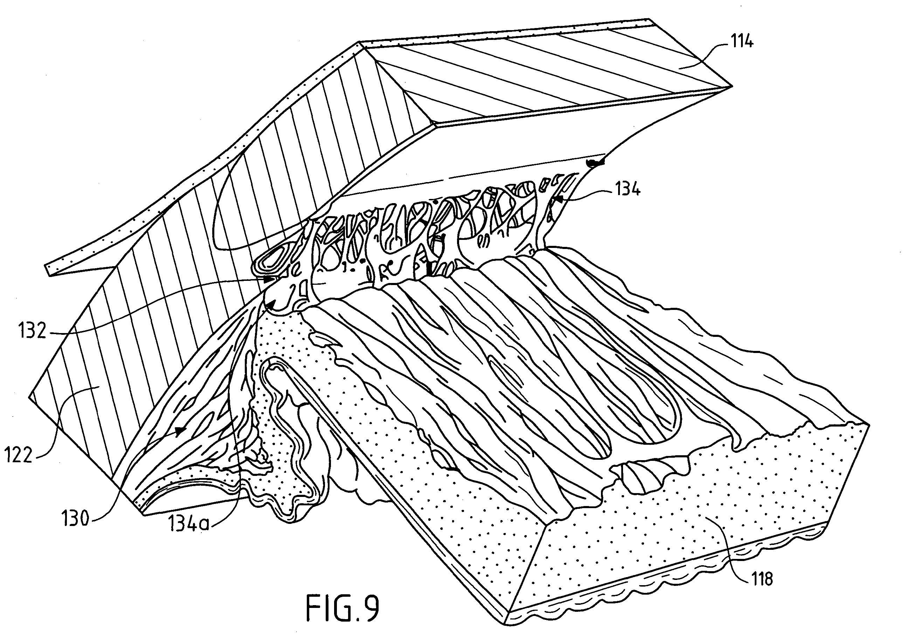

Un implant 140 selon un mode de réalisation de l'invention a été interposé entre la sclère 122 et le muscle ciliaire 130 comme décrit ci-dessus. Cet implant est positionné le plus près possible du trabeculum (grâce à son bord antérieur concave) afin d'exercer son effet d'écartement permanent à l'endroit le plus approprié, tout en respectant l'insertion du muscle ciliaire 130 à l'éperon scléral. La figure 9 est une vue plus détaillée et agrandie de la structure de l'angle iridocornéen sans l'implant. Comme représenté sur cette figure, l'éperon scléral 132 sur lequel est inséré le muscle ciliaire 130 est situé au dessus de la partie postérieure 134a du trabeculum 134.

L'écartement produit à cet endroit entre la sclère et le corps ciliaire permet de recueillir de manière permanente l'humeur aqueuse au plus près de la zone de l'écoulement physiologique uvéo-scléral (l'effet d'écartement crée une zone de moindre résistance à l'écoulement de l'humeur aqueuse). Un tel implant ainsi positionné apporte un gain significatif dans l'augmentation de l'écoulement uvéo-scléral physiologique.

L'écoulement physiologique uvéo-scléral est accru par une fraction supplémentaire d'écoulement à travers la partie postérieure du trabeculum (trabeculum ciliaire), comme représenté sur la figure 8 par les flèches F5 situées au dessus et en dessous de l'implant. L'écoulement de cette fraction supplémentaire est obtenu grâce à l'effet d'écartement de l'implant entre la sclère et le corps ciliaire, au plus près du trabeculum, sans toutefois endommager ce dernier.

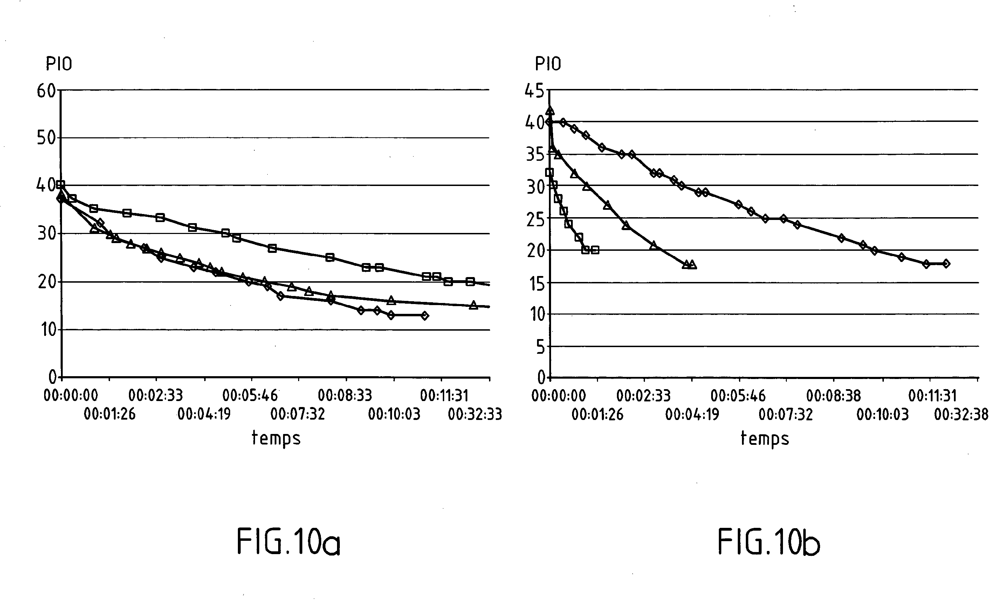

Les figures 10a et 10b illustrent les résultats d'une expérience réalisée sur un oeil de banque des yeux ne pouvant être utilisé pour des greffes de cornée.

Un poids de 15g correspondant à une pression intra-oculaire (PIO) simulant les conditions d'un glaucome a été posé sur le sommet de la cornée de l'œil.

Un capteur de pression a été placé à l'intérieur de l'œil pour mesurer la PIO. Des mesures successives de PIO (tonographie) ont été enregistrées sur l'œil sans implant, puis avec implant, afin de mesurer un effet éventuel de l'implant sur l'écoulement de l'humeur aqueuse, et donc sur la PIO.

Un implant conforme à l'invention, notamment à un mode de réalisation de l'invention, a été mis en place dans l'oeil à travers deux incisions sclérales suivant la méthode d'implantation chirurgicale décrite en référence à la figure 7. L'implant a été introduit dans l'espace supra-ciliaire.

L'implant en question est un implant de dimensions 5x3mm, de 150 µm d'épaisseur, réalisé en matériau acrylique hydrophile à 25%, connu pour son uvéo-compatibilité. La forme de l'implant est celle de l'implant 30 de la figure 1.

Des mesures successives de la PIO sont réalisées et enregistrées (en mm Hg), ici pendant 12 mn, afin d'identifier la facilité d'écoulement de l'humeur aqueuse. Cette technique est connue sous le nom de tonographie. Cette technique est par exemple décrite dans la référence suivante : J Glaucoma. 2003 Jun ;12(3) :237-42 « Tonography demonstrates reduced facility of outflow of aqueous humor in myocilin mutation carriers », Wilkinson CH1, Van der Straaten D, Craig JE, Coote MA, McCartney PJ, Stankovitch J, Stone EM, Mackey DA.

Différentes courbes de résultats de mesures illustrées sur la figure 10a (courbes comportant des carrés, des triangles et des losanges) sont celles obtenues sur l'oeil sans implant en exerçant différentes pressions locales sur l'œil. Les pressions différentes exercées simulent les variations de la PIO telles qu'on peut en rencontrer dans le glaucome et les conséquences sur l'écoulement. Les deux courbes avec les carrés et les triangles sont celles pour lesquelles des pressions locales ont été exercées.

Différentes courbes de résultats de mesures illustrées sur la figure 10b (courbes comportant des carrés, des triangles et des losanges) sont celles obtenues sur l'œil avec l'implant précité en exerçant différentes pressions locales sur l'œil (courbes avec carrés et triangles).

La comparaison des courbes correspondantes entre les deux figures montre que les courbes de la figure 10b ont des pentes supérieures (gradient de PIO plus grand) à celles de la figure 10a et ainsi que le temps est plus court pour que la PIO revienne à une pression physiologique située entre 10 et 20 mmHg. Le rétablissement plus rapide d'une PIO physiologique avec un implant conforme à l'invention signifie que l'écoulement d'humeur aqueuse a été amélioré de manière très significative.

L'inversion des courbes entre les figures 10a et 10b tient à ce que, en présence de l'implant, l'écoulement est plus important avec une pression exercée plus élevée.

## Revendications

1. Implant ophtalmologique d'interposition permanente entre la sclère et le tissu uvéal, l'implant comprend un corps (20 ;30 ;40 ;50) uvéocompatible, formé d'une seule partie, le corps mince comportant trois dimensions dans l'espace, à savoir une longueur et une largeur qui sont perpendiculaires à une épaisseur, le corps mince ayant une seule épaisseur (e) sensiblement uniforme qui est au moins inférieure à 10 fois la plus petite des deux autres dimensions du corps, le corps de l'implant comprenant deux bords opposés (22,24 ;32,34 ;42 ;44 ;52 ,54) qui sont éloignés l'un de l'autre suivant une des deux dimensions perpendiculaires à l'épaisseur, l'un des bords appelé bord antérieur (22 ;32 ;42 ;52) présentant, en projection dans un plan perpendiculaire à l'épaisseur, une courbure concave tournée vers l'extérieur du corps, les deux bords opposés (22,24 ;32,34 ;42 ;44 ;52 ,54) du corps, à savoir le bord antérieur et le bord opposé postérieur, étant éloignés l'un de l'autre suivant la plus petite des deux dimensions perpendiculaires à l'épaisseur, la courbure concave du bord antérieur permettant de positionner l'implant au plus près du trabeculum pour recueillir de manière permanente l'humeur aqueuse au plus près de la zone de l'écoulement uvéo-scéral.

2. Implant ophtalmologique d'interposition selon la revendication 1, **caractérisé en ce que** le bord antérieur concave (22 ;32 ;42 ;52) est aminci sur une dimension qui est comprise entre 100 et 400µm, cette dimension étant prise suivant une direction qui s'étend entre les deux bords opposés du corps.

3. Implant ophtalmologique d'interposition selon l'une des revendications précédentes, **caractérisé en ce que** la courbure concave du bord antérieur (22 ;32 ;42 ;52) présente un rayon de courbure compris entre 5 et 7 mm.

4. Implant ophtalmologique d'interposition selon l'une des revendications précédentes, **caractérisé en ce que** le corps de l'implant (50) est élastiquement déformable de manière à pouvoir être plié sans induire de déformation permanente pour être manipulé avec un micro instrument, ou injecté avec un système d'injection ophtalmologique.

5. Implant ophtalmologique d'interposition selon la revendication 4, **caractérisé en ce que** le corps de l'implant (50) comporte, dans un état déformé dans lequel il est susceptible d'être utilisé comme implant ophtalmologique d'interposition entre la sclère et le tissu uvéal, une deuxième courbure concave dans une direction perpendiculaire à un plan défini par les deux dimensions de l'implant qui sont perpendiculaires à l'épaisseur (e).

6. Implant ophtalmologique d'interposition selon l'une des revendications précédentes, **caractérisé en ce que** le corps de l'implant est réalisé dans un matériau qui possède un module de Young compris entre 30 et 60 kg/cm2.

7. Implant ophtalmologique d'interposition selon l'une des revendications 1 à 3, **caractérisé en ce que** le corps de l'implant n'est pas élastiquement déformable et comporte, de manière permanente, une autre courbure concave dans une direction perpendiculaire à un plan défini par les deux dimensions de l'implant qui sont perpendiculaires à l'épaisseur.

8. Implant ophtalmologique d'interposition selon l'une des revendications précédentes, **caractérisé en ce que** le corps de l'implant possède deux grandes faces opposées (60a,60b ;72a,72b ;82a,82b) écartées l'une de l'autre suivant l'épaisseur du corps.

9. Implant ophtalmologique d'interposition selon la revendication 8, **caractérisé en ce que** le corps de l'implant est percé d'orifices (62 ;89) traversant son épaisseur ou le corps de l'implant comprend sur au moins une de ses deux grandes faces opposées (72a,72b ;82a,82b) un relief (72,74 ;82,84) qui est apte à favoriser un écoulement de l'humeur aqueuse le long de ladite au moins une grande face.

10. Implant ophtalmologique d'interposition selon la revendication 9, **caractérisé en ce que** le relief de ladite au moins une grande face du corps prend la forme de rainures (72,74 ;82,84) aménagées sur ladite au moins une grande face ou d'une rugosité conférée à celle-ci.

11. Implant ophtalmologique d'interposition selon l'une des revendications précédentes, **caractérisé en ce que** le corps de l'implant comprend au moins un matériau qui est choisi parmi les matériaux suivants : le PTFE, le polysiloxane, les hydrogels acrylates hydrophiliques ou hydrophobiques.

12. Implant ophtalmologique d'interposition selon l'une des revendications précédentes, **caractérisé en ce que** l'épaisseur (e) du corps de l'implant est comprise entre 50 et 400µm.

13. Implant ophtalmologique d'interposition selon l'une des revendications précédentes, **caractérisé en ce que** le corps de l'implant présente des propriétés de relargage d'une ou de plusieurs substances.

14. Implant ophtalmologique d'interposition selon l'une des revendications précédentes, **caractérisé en ce que** le corps présente en projection dans un plan perpendiculaire à l'épaisseur un contour périphérique externe à géométrie convexe, à l'exception de la portion du corps qui comporte le bord antérieur concave.

15. Implant ophtalmologique d'interposition selon l'une des revendications précédentes, **caractérisé en ce que** l'autre des deux bords opposés est appelé bord postérieur, le bord postérieur étant éloigné du bord antérieur d'une distance d'au moins 3 mm.

## Patentansprüche

1. Permanentes interpositionelles ophthalmologisches Implantat zwischen der Sklera und dem uvealen Gewebe, wobei das Implantat einen mit der Uvea kompatiblen Körper (20; 30; 40; 50) umfasst, der aus einem einzigen Teil gebildet ist, wobei der dünne Körper drei Dimensionen im Raum aufweist, d. h. eine Länge und eine Breite, die senkrecht zu einer Dicke sind, wobei der dünne Körper eine einzige im Wesentlichen gleichförmige Dicke (e) aufweist, die mindestens weniger als 10 Mal die kleinste der zwei anderen Dimensionen des Körpers ist, wobei der Körper des Implantats zwei einander gegenüberliegende Ränder (22, 24; 32, 34; 42; 44; 52, 54) aufweist, die voneinander gemäß einer der zwei senkrechten Dimensionen zur Dicke entfernt sind, wobei einer der Ränder, bezeichnet als vorderer Rand (22; 32; 42; 52), in Projektion in einer senkrechten Ebene zur Dicke eine konkave Krümmung aufweist, die hin zur Außenseite des Körpers gedreht ist, wobei die zwei einander gegenüberliegenden Ränder (22, 24; 32, 34; 42; 44; 52, 54) des Körpers, d. h. der vordere Rand und der gegenüberliegende hintere Rand, voneinander gemäß der kleinsten der zwei Dimensionen senkrecht zur Dicke entfernt sind, wobei die konkave Krümmung des vorderen Rands ermöglicht, das Implantat so nahe wie möglich am Trabekel zu positionieren, um auf permanente Weise das Augenkammerwasser zu sammeln, das dem Bereich des Uvea-Sklera-Ablaufs am nächsten ist.

2. Interpositionelles ophthalmologisches Implantat nach Anspruch 1, **dadurch gekennzeichnet, dass** der vordere konkave Rand (22; 32; 42; 52) auf einer Dimension verdünnt ist, die im Bereich zwischen 100 und 400 µm liegt, wobei diese Dimension gemäß einer Richtung genommen wird, die sich zwischen den zwei einander gegenüberliegenden Rändern des Körpers erstreckt.

3. Interpositionelles ophthalmologisches Implantat nach einem der vorhergehenden Ansprüche, **dadurch gekennzeichnet, dass** die konkave Krümmung des vorderen Rands (22; 32; 42; 52) einen Krümmungsradius darstellt, der im Bereich zwischen 5 und 7 mm liegt.

4. Interpositionelles ophthalmologisches Implantat nach einem der vorhergehenden Ansprüche, **dadurch gekennzeichnet, dass** der Körper des Implantats (50) elastisch verformbar ist, so dass er gefaltet werden kann, ohne zu einer dauerhaften Verformung zu führen, um mit einem Mikroinstrument gehandhabt oder mit einem ophthalmologischen Injektionssystem injiziert zu werden.

5. Interpositionelles ophthalmologisches Implantat nach Anspruch 4, **dadurch gekennzeichnet, dass** der Körper des Implantats (50) in einem verformten Zustand, in dem es wahrscheinlich ist, dass er als interpositionelles ophthalmologisches Implantat zwischen der Sklera und dem Uvea verwendet wird, eine zweite konkave Krümmung in einer Richtung senkrecht zu einer Ebene aufweist, die von den zwei Dimensionen des Implantats definiert wird, die senkrecht zu der Dicke (e) sind.

6. Interpositionelles ophthalmologisches Implantat nach einem der vorhergehenden Ansprüche, **dadurch gekennzeichnet, dass** der Körper des Implantats aus einem Material hergestellt ist, das ein Young'sches Modul im Bereich zwischen 30 und 60 kg/cm² aufweist.

7. Interpositionelles ophthalmologisches Implantat nach einem der Ansprüche 1 bis 3, **dadurch gekennzeichnet, dass** der Körper des Implantats nicht elastisch verformbar ist und auf permanente Weise eine weitere konkave Krümmung in einer Richtung senkrecht zu einer Ebene umfasst, die von den zwei Dimensionen des Implantats definiert ist, die senkrecht zu der Dicke sind.

8. Interpositionelles ophthalmologisches Implantat nach einem der vorhergehenden Ansprüche, **dadurch gekennzeichnet, dass** der Körper des Implantats zwei große einander gegenüberliegende Flächen (60a, 60b; 72a, 72b; 82a, 82b) aufweist, die voneinander gemäß der Dicke des Körpers beabstandet sind.

9. Interpositionelles ophthalmologisches Implantat nach Anspruch 8, **dadurch gekennzeichnet, dass** der Körper des Implantats von Öffnungen (62; 89) durchbrochen ist, die seine Dicke durchqueren, oder der Körper des Implantats auf mindestens einer seiner zwei großen einander gegenüberliegenden Flächen (72a, 72b; 82a, 82b) ein Relief (72, 74; 82, 84) aufweist, das dazu in der Lage ist, ein Ablaufen der Augenkammerwassersentlang der mindestens einen großen Fläche zu begünstigen.

10. Interpositionelles ophthalmologisches Implantat nach Anspruch 9, **dadurch gekennzeichnet, dass** das Relief der mindestens einen großen Fläche des Körpers die Form von Nuten (72, 74; 82, 84), die auf der mindestens einen großen Fläche angeordnet sind, oder einer Rauigkeit, die dieser zugewiesen ist, annimmt.

11. Interpositionelles ophthalmologisches Implantat nach einem der vorhergehenden Ansprüche, **dadurch gekennzeichnet, dass** der Körper des Implantats mindestens ein Material umfasst, das aus den folgenden Materialien ausgewählt ist: PTFE, Polysiloxan, hydrophilen oder hydrophoben Acrylathydrogelen.

12. Interpositionelles ophthalmologisches Implantat nach einem der vorhergehenden Ansprüche, **dadurch gekennzeichnet, dass** die Dicke (e) des Körpers des Implantats im Bereich zwischen 50 und 400 µm liegt.

13. Interpositionelles ophthalmologisches Implantat nach einem der vorhergehenden Ansprüche, **dadurch gekennzeichnet, dass** der Körper des Implantats Eigenschaften der Freisetzung einer oder mehrerer Substanzen aufweist.

14. Interpositionelles ophthalmologisches Implantat nach einem der vorhergehenden Ansprüche, **dadurch gekennzeichnet, dass** der Körper in Projektion auf einer Ebene senkrecht zur Dicke einen äußeren peripheren Umfang mit konvexer Geometrie aufweist, mit Ausnahme des Abschnitts des Körpers, der den vorderen konkaven Rand umfasst.

15. Interpositionelles ophthalmologisches Implantat nach einem der vorhergehenden Ansprüche, **dadurch gekennzeichnet, dass** der andere der zwei gegenüberliegenden Ränder als hinterer Rand bezeichnet wird, wobei der hintere Rand vom vorderen Rand um eine Distanz von mindestens 3 mm beabstandet ist.

## Claims

1. A permanent interpositional ophthalmological implant between the sclera and the uveal tissue, the implant comprises a body (20; 30; 40; 50) with uveal compatibility, formed as a single part, the thin body having three dimensions in space, namely a length and a width which are perpendicular to a thickness, the thin body having a substantially uniform single thickness (e) which is at least less than 10 times the smaller of the other two dimensions of the body, the body of the implant comprising two opposite edges (22, 24; 32, 34; 42; 44; 52, 54) which are distant from one another along one of the two dimensions perpendicular to the thickness, one of the edges, called anterior edge (22; 32; 42; 52), having, in projection in a plane perpendicular to the thickness, a concave curvature facing toward the outside of the body, the two opposite edges (22, 24; 32, 34; 42; 44; 52, 54) of the body, namely the anterior edge and the opposite posterior edge, being distant from one another along the smaller of the two dimensions perpendicular to the thickness, the concave curvature of the anterior edge allowing the implant to be positioned as close as possible to the trabecular meshwork in order to permanently collect the aqueous humor as close as possible to the uveoscleral flow.

2. The interpositional ophthalmological implant according to claim 1, **characterized in that** the concave anterior edge (22; 32; 42; 52) is thinned over a dimension of between 100 and 400 µm, this dimension being considered in a direction extending between the two opposite edges of the body.

3. The interpositional ophthalmological implant according to one of the preceding claims, **characterized in that** the concave curvature of the anterior edge (22; 32; 42; 52) has a radius of curvature of between 5 and 7 mm.

4. The interpositional ophthalmological implant according to one of the preceding claims, **characterized in that** the body of the implant (50) is elastically deformable so that it can be bent without inducing permanent deformation so that it can be handled using a micro instrument, or injected using an ophthalmological injection system.

5. The interpositional ophthalmological implant according to claim 4, **characterized in that** the body of the implant (50) comprises, in a deformed state in which it is capable of being used as an interpositional ophthalmological implant between the sclera and the uveal tissue, a second concave curvature in a direction perpendicular to a plane defined by the two dimensions of the implant which are perpendicular to the thickness (e).

6. The interpositional ophthalmological implant according to one of the preceding claims, **characterized in that** the body of the implant is made from a material which has a Young's modulus of between 30 and 60 kg/cm².

7. The interpositional ophthalmological implant according to one of claims 1 to 3, **characterized in that** the body of the implant is not elastically deformable and permanently comprises another concave curvature in a direction perpendicular to a plane defined by the two dimensions of the implant which are perpendicular to the thickness.

8. The interpositional ophthalmological implant according to one of the preceding claims, **characterized in that** the body of the implant has two large opposite faces (60a, 60b; 72a, 72b; 82a, 82b) separated from one another along the thickness of the body.

9. The interpositional ophthalmological implant according to claim 8, **characterized in that** the body of the implant is pierced with orifices (62; 89) passing through its thickness or the body of the implant comprises, on at least one of its two large opposite faces (72a, 72b; 82a, 82b), a relief (72, 74; 82, 84) which is able to encourage a flow of aqueous humor along said at least one large face.

10. The interpositional ophthalmological implant according to claim 9, **characterized in that** the relief on said at least one large face of the body takes the form of grooves (72, 74; 82, 84) formed on said at least one large face or of roughness conferred thereon.

11. The interpositional ophthalmological implant according to one of the preceding claims, **characterized in that** the body of the implant comprises at least one material chosen from the following materials: PTFE**,** polysiloxane, hydrophilic or hydrophobic acrylate hydrogels.

12. The interpositional ophthalmological implant according to one of the preceding claims, **characterized in that** the thickness (e) of the body of the implant is between 50 and 400 µm.

13. The interpositional ophthalmological implant according to one of the preceding claims, **characterized in that** the body of the implant has properties of releasing one or more substances.

14. The interpositional ophthalmological implant according to one of the preceding claims, **characterized in that** the body has, in projection in a plane perpendicular to the thickness, an external peripheral contour with a convex geometry, with the exception of the portion of the body which comprises the concave anterior edge.

15. The interpositional ophthalmological implant according to one of the preceding claims, **characterized in that** the other of the two opposite edges is called the posterior edge, the posterior edge being distant from the anterior edge by a distance of at least 3 mm.
